# EUROPEAN PATENT APPLICATION

(11) **EP 4 265 251 A1**
(43) Date of publication of application: **25.10.2023**
(21) Application number: 22305577.3
(22) Date of filing: 20.04.2022
(51) Int. Cl.: A61K 31/46, A61K 9/06, A61K 47/36, A61K 47/38, A61K 47/12, A61K 9/00

(54) **NEW FORMULATION OF ATROPINE**

(71) Applicant: Assistance Publique Hôpitaux de Paris, 75004 Paris (FR); Université Paris Cité, 75006 Paris (FR)
(72) Inventor: The designation of the inventor has not yet been filed
(74) Representative: Flesselles, Bruno F.G.

(57) **Abstract**

The invention pertains to a new formulation of atropine in the form of a gel, in particular for use for the treatment of treatment of sialorrhea.

## Description

The invention pertains to a new formulation of atropine in the form of a gel, in particular for use for the treatment of treatment of sialorrhea, in particular for patients with neurological diseases/disorders, notably secondary to Parkinson's disease, or for neurologically impaired children.

Sialorrhea, which is also known as drooling, is excessive salivation, which is generally associated with neurological disorders but also to anatomical abnormalities of the oral cavity or dysphagia. Such neurologic disorders include cerebral palsy (in particular for children), Parkinson's disease, and amyotrophic lateral sclerosis, or as an adverse effect of medications (such as clozapine).

Sublingual administration of atropine has been proposed for treatment of sialorrhea for children (Rapoport, J Pain Symptom Manage., 2010;40(5):783-8), following clozapine treatment (Van der Poorten and De Hert, Clin Case Rep. 2019; 7(11): 2108-2113) or for Parkinson patients (Hyson et al, Mov Disord, 2002;17(6):1318-20).
Atropine is currently used as eye drops administered via sublingual route.In clinic, atropine is currently used in form of eye drops administrated sublingually at the dose of one or two drops based on the patients symptoms. Therefore there is a major risk of toxicity due to under or overdosing since the actual packaging doesn't have a precise dropper advice. Furthermore, the bitter taste of atropine may reduce the acceptability of the treatment for the patients.

There is thus a need to provide new formulations of atropine that would be easier to administer, with a better control of the dosage, and advantageously with improved onsite delivery (with an increased time in the patient's mouth). Such formulations shall be applied sublingually, in order to allow the most appropriate way of administering atropine, as it prevents passage within the gastric environment and improves the therapeutic effect of atropine, as the active substance would pass directly from the sublingual mucosa to the blood circulation. Furthermore, it is preferred that the formulation stay in the mouth long enough for atropine to enter blood circulation, without being eluted by saliva.

. This formulation offers several advantages:
a) it is easier to apply than the current drop by drop formulation of atropine eye-solution.
b) It provides a better control of the dose administered to the patient, as the volume can be easily determined prior to the sublingual administration, whereas the drop by drop administration presents a risk of over-dosing. Such better control increases the patient's safety.
c) It makes it possible to increase the exchange surface in the buccal cavity by depositing the gel on the buccal mucosa a on larger surface that where a drop would fall, especially sublingually, and
d) In some embodiments, it could be used for patients with a tracheostomy cannula.
e) It makes it possible to increase the amount of time the active ingredient is present in the buccal cavity by virtue of its gel form; it makes it possible to obtain better bioavailability
f) Using appropriate buffers, sweeteners, flavors or aromas, or masking agents, it makes it possible to increase patient's acceptability and compliance, by neutralizing or masking bitterness of atropine.

The invention this relates to a composition in the form of a gel, comprising:
a) Atropine
b) A thickening agent providing viscosity of the composition
c) A buffer for stabilizing the pH of the composition between 3.5 and 4.5.

The composition is in the form for oral administration, and is intended to be provided under the tongue of the patient (sublingual administration), either by direct deposit, or by spraying, depending on the form of the application system.

The thickening agent provides the viscosity of the formulation, allowing it to have the form of a gel. It is reminded that a gel is a three dimensional matrix composed of two interpenetrating systems, the thickening agent or gelator, and the dispersion medium comprising the active principle (here atropine) in solution.

The gel's viscosity should be adapted for an oral administration (in particular for a sublingual administration). It is thus preferred when the viscosity is comprised between 200 and 1000 mPa.s, preferably between 200 and 600 mPa.s, more preferably between 250 and 400 mPa.s at 25°C). Due to the fact that the composition should be stored at room temperature and used within the human body, it it preferred when it presents a viscosity that would be between 200 and 1000 mPa.s between 20°C and 37°C. Viscosity is measured according to methods known in the art. In particular, the rheological analysis wan be carried out using an Anton Paar MCR 500 rheometer (Courtaboeuf, France), equipped with a cone plate combination (diameter = 50 mm; angle = 1°) as measuring system. This method is preferred, also other modules (8 mm-150 mm, angles from 0-5°) could also be used. The apparent viscosity (Pa·s) of the formulation can also be measured with a continuous shear (flow) model.

The thickening agent is selected to be compatible for pharmaceutical uses. In particular, it is advantageous to select it from the group consisting of hydroxyethyl cellulose, hydroxypropylmethyl cellulose, carboxymethyl cellulose, polyvinyl alcohol, gum Arabic, guar gum, locust bean gum, gellan gum, carrageenan, xanthan gum and carob.

Polymers based on cellulose are preferred, in particular hydroxyethyl cellulose or hydroxypropylmethyl cellulose. Hydroxyethyl cellulose is particularly adapted and is generally used at a concentration between 2 and 4 % (w/w), more preferably between 2.5 and 3.5 % (w/w). An amount of about 3% (w/w in the composition) is particularly suitable.

The pH of the composition is acidic, in order to stabilize atropine. This also serves to mask the bitter taste of it. It is of particular interest to use a buffer for stabilizing the pH of the composition between 3.5 and 4.5. Any buffer suitable for pharmaceutical use can be chosen. In particular, one can select the buffer from the group consisting of citrate buffer, phosphate buffer, citrate-phosphate (Mcllvaine) buffer and acetate buffer. Citrate buffer is of particular note as it also provides a citrus taste. Such taste is known and contributes to increase the acceptability by the patient when the composition is administered.

In some embodiments, the composition also comprises at least one agent capable of inducing, strengthening or improving the muco-adhesion or the gelling upon buccal administration, in particular upon sublingual administration. This agent may increase the viscosity of the composition in particular being in the liquid state when present in the composition and gelling when in contact with the mucous membranes of the buccal cavity, in particular the sublingual mucous membranes.

One can use an agent capable of interacting with the divalent cations that are present in the saliva or on the surface of the mucous membrane of the mouth. In particular sodium alginate gels when entering in contact with Ca²⁺ cations that are present in the mouth. Gelling of sodium alginate increases the viscosity of the composition after application in the mouth (preferably under the patient's tongue) which extends the length of stay of the composition, and thus improves the passing of atropine within the circulation through the mucosa.

One can also use pectins, in particular with a low degree of methylation, which are also capable of gelling in contact of Ca²⁺ cations in acidic conditions.

In another embodiment, the agent capable of inducing or of strengthening the muco-adhesion or the gelling upon buccal administration comprises a poloxamer or a mixture of poloxamers. It is reminced that a poloxamer is refers to a tri-block copolymer comprising or consisting of a central polyoxypropylene chain (also called polypropylene glycol, PPO) grafted on either side by a chain of polyoxyethylene (also known as polyethylene glycol, POE). Poloxamers are generally denoted by the letter "P" (for poloxamer) followed by three numbers: the first two numbers multiplied by 100 give the molecular weight of the polyoxypropylene core, and the final number multiplied by 10 gives the percentage polyoxyethylene content.

Useful poloxamers are ones that are in the liquid state at room temperature, and in particular between 2°C and 25°C, and in the state of gel at a temperature greater than or equal to the gelling temperature (Tg), especially under physiological conditions, and in particular between 30°C and 40°C. Temperatures of gelation of poloxamers (Tg) can be determined according to conventional methods as explained above or are available in reference works such as the Handbook of Pharmaceutical Excipients. These poloxamers are present in a concentration sufficient in the gel solution of atropine to increase the viscosity thereof when the temperature is greater than or equal to their gelling temperature (T_{g}), in particular under physiological conditions (at around 37 °C). Thus, and preferably, the poloxamer gels at a temperature between 30°C and 37°C, more preferentially on contact with mucosa of the mouth. One can note that, although the sublingual administration is preferred, one also envisage to spray the composition, when containing a poloxamer onto the mucous membranes of the cheeks.

Poloxamer 407, poloxamer 188 or mixtures thereof are particularly adapted.

The composition may also contain at least one further component selected from the group consisting of aromas, flavors, dyes, sweetener and masking agent. These components are used to improve palatability of the composition (and mask the bitter taste of atropine), and also improve its visual aspect.

One can cite low-calorie sweeteners such as sucralose, and stevia or extracts thereof (such as rebaudioside A at 97%). Taste masking agents may be chosen from natural and synthetic flavor liquids. Flavors useful include, without limitation, volatile oils, synthetic flavor oils, flavoring aromatics, oils, liquids, oleoresins or extracts derived from plants, leaves, flowers, fruits, stems and combinations thereof. A non-limiting list of examples include citrus oils (such as lemon, orange, grape, lime and grapefruit) and fruit essences including apple, pear, peach, grape, strawberry, raspberry, cherry, plum, pineapple, apricot or other fruit flavors. One can also use caramel or cola flavors. The flavor is preferably associated with an acidic taste so as to be consistent with the pH of the solution. Citrus flavor, in particular lemon or lime flavor is favored when citrate buffer is used to stabilize the pH.

A preservative may also be added to the composition, especially when the formulation is packaged in a multi-dose container. One uses preferably a paraben-based preservative, such as methyl-paraben, ethyl-paraben, propyl-paraben or butyl-paraben, although other preservatives such as benzyl alcohol, cresols, benzoic acid or sodium benzoate, sodium metabisulfite, propylene glycol, ethanol, phenol, or sorbic acid and its salts can also be envisaged.

It may also be interesting to add agents that help solubilizing the preservatives, in particular when paraben-based preservatives are used. One can cite non-ionic surfactants (such as poloxamer 407 or 188), Tocofersolan (tocopherol polyethylene glycol succinate, TPGS, a synthetic water-soluble version of vitamin E), diethylene glycol monoethyl ether, propylene glycol, cyclodextrins, or ionic surfactants such as polysorbates.

In one embodiment, the composition is packaged as a single unit dose, which contains from 100 a 2000 µg (0.1 to 2 mg) atropine. The design of the single unit dose is adapted for sublingual administration of atropine. The value ranges in the form "from x to y" include the limits x and y. In particular, the single unit dose may contain between from 400 to 600 µg atropine, in particular about 500 µg. The volume of the single unit dose is preferably about 0.2 mL.

In another embodiment, the composition is packaged in a kit, which also comprises a syringe dose dropper adapted for viscous formulation. This syringe dose dropper comprises graduations so that the amount of the composition taken from the container and administered to the patient can be easily controlled. The syringe can easily be placed below the patient's tongue for sublingually administering the composition.

In another embodiment, the composition is packaged as a spray, wherein a dose from 100 to 2000 µg (0.1 to 2 mg) atropine is delivered for each spray, more specifically from 400 to 600 µg (in particular about 500 µg). Alternatively, this dosage of atromine may be delivered with two or more sprays. Since the composition is already in the form of a gel, in this embodiment, the viscosity of the gel may be reduced (in the lower ranges indicated above) as compared to the viscosity for composition that are to be deposited directly on the sublingual mucosa. The device for spraying the composition can also be adapted to be able to nebulize the gel composition. In this embodiment, it is preferred when the composition contains a poloxamer as described above, for instantaneously forming a thicker gel on the area of application, which is preferentially sublingual.

The invention also relates to a method for preparing or making a composition herein disclosed comprising
a) Preparing a solution of atropine in a buffer so as to obtain a solution with a pH between 3.5 and 4.5
b) Optionally adding aromas, flavors, dyes, sweetener and masking agent
c) Optionally adding an agent capable of improving muco-adhesion upon buccal administration
d) Optionally adding a preservative
e) Adding the thickening agent,
so as to obtain a composition in the form of a gel, comprising atropine, and with the pH stabilized between 3.5 and 4.5.

Addition of the components in b), c), d), and e) is preferably performed under stirring, in order to improve and insure good dissolution of the components. It is particularly advisable to add the thickening agent in e) under stirring.

The speed of stirring is adapted to the viscosity of the composition, when adding the thickening agent. In particular, said agent is added progressively to avoid formation of lumps and bad solubilization, even though it is a soluble component. This is generally also true for the components that are in the form of powders. The composition preferably contains from 0.15 to 0.35% (w/w) atropine, more preferably from 0.2 to 0.3%, more preferably from 0.2 to 0.25%.

The amount of the thickening agent is determined according to the specific properties of the agent used in the formulation, so as to obtain the desired rheology of the composition (gel composition that can easily be deposited under the tongue and remain there). The composition obtained or susceptible to be obtained by this method (and by methods of the examples) is also part of the invention.

According to one embodiment, the formulations of the invention comprise approximately 0.25% (w/w) atropine, approximately 3% (w/w) hydroxyethyl cellulose, approximately 0.25% (w/w) sodium alginate, approximately 0.12% (w/w) sweetener (notably sucralose), optionally approximately 0.12% w/w preservatives (notably propyl-paraben) and qsp 100% citrate buffer.

The term "approximately" refers to a range of values of ± 5% of a specific value.

The formulations are preferably sterile and can be prepared by sterilizing filtration, by radiosterilization, or else under aseptic conditions. More particularly, this solution are prepared and/or stored under sterile conditions in the appropriate containers (single dose container, bottle, optionally equipped with a suitable sprayer).

The invention also relates to a pharmaceutical composition comprising or consisting of a gel formulation as defined above. This formulation is provided by the oral route, preferably sublingually, or through spaying under the tongue or ob the mucosa of the cheeks.

The invention also relates to a composition herein disclosed for use thereof for the treatment of sialorrhea, intended in particular to be administered in the buccal cavity, sublingually. A dose of 500 µg atropine is perfectly adapted for such uses.

As indicated, sialorrhea can be treated in patients suffering from neurological diseases, in particular Parkinson disease, ALS (amyotrophic lateral sclerosis), multiple sclerosis or sequelae after a stroke.

### EXAMPLES

### Example 1. Preparation of a gel formulation of atropine

A stock aqueous solution of atropine sulfate of 2% m/m which was 8 times more concentrated than the final product was prepared.

As a result, a storage solution was obtained whose chemical stability was evaluated at at least 21 days. This solution was stored at 4°C.

Citrate buffer was used as the solvent for the formula, in order to maintain the pH at 4. The pH was verified before each use.

For preparation of development batches (20 g), the preparations were made under agitation using a magnetic bar of a size adapted to the container. The step consisted in diluting a small volume of the stock solution of atropine sulfate in a defined volume of citrate buffer in order to obtain a solution 2 of atropine sulfate with a final concentration in atropine of 0.25%. The obtained solution was homogenized under stirring.

In order to sweeten the solution for oral use, sucralose was then added in solution 2, as it is an easily soluble substance. Its solubilization was fast, without the need to increase the stirring speed. No physically visible incompatibility was observed. Solution 3 was then obtained.

The following step was the solubilization of sodium alginate. Sodium alginate is a natural polymer whose gelation is obtained in the presence of calcium ions. In the absence of calcium, the sodium alginate solution remains liquid. Since solution 3 did not contain calcium ions, the dissolution of sodium alginate was relatively simple, although it was carried out delicately (by the "fine rain" method) because residual solid particles can remain. The addition of the total amount of polymer, even if under agitation, can induce the appearance of lumps difficult to dissolve. Therefore alginate sodium (in the form of a powder) was added little by little in order to avoid the agglomeration of the powder. The agitation was maintained throughout the step. Once solution 4 was obtained, no incompatibility was visually observed.

Once sodium alginate was dissolved, Hydroxy Ethyl Cellulose (HEC) was added to the formula. Unlike alginate, the addition of HEC induces the increase of the viscosity of the formula. The solubilization of this polymer required an adjustment of the stirring speed to ensure a permanent vortex. Being a water soluble substance, the solubilization of HEC was performed at room temperature. Increasing the heat to initiate or improve solubilization is also suitable. However, care should be taken so as to avoid potential degradation of the active ingredient. The HEC powder was added progressively "in a fine rain" in order to limit the formation of lumps as much as possible, while constantly adjusting the stirring speed according to the viscosity of the solution.

The preparation was stored at 4°C overnight to ensure complete hydration of the polymeric chains of the HEC. Routine tests included pH control, rheological analysis and mucoadhesive ability analysis.

### Example 2. Use of a gel formulation of atropine

An acceptability/palatability test of the formulation was performed for 4 patients. The results show that:
- 2 patients find a "pleasant" taste of the new formula, 1 patient a "neutral" taste and only one patient found a taste considered "unpleasant", in particular as a flavored taste was expected.

The four patients confirmed that the taste was different from the eye drops formulation's taste, in particular as being much less bitter.

One patient indicated that the taste was better and lasted longer, which would suggest a longer stay in the mouth.

On the caregivers' side, the feedback is also positive.

The two nurses found the preparation and administration methods easier than the administration of the eye drops, as it allows better control of the dose and makes it easier to administer under the tongue.

## Claims

1. Composition in the form of a gel, comprising:
a) Atropine
b) A thickening agent providing viscosity of the composition
c) A buffer for stabilizing the pH between 3.5 and 4.5.

2. The composition of claim 1, which is in the form for oral administration.

3. The composition of claim 1 or 2, wherein the thickening agent is selected from the group consisting of hydroxyethyl cellulose, hydroxypropylmethyl cellulose carboxymethyl cellulose, polyvinyl alcohol, gum Arabic, guar gum, locust bean gum, gellan gum, carrageenan, xanthan gum and carob.

4. The composition of claim 1 to 3, wherein the buffer is selected from the group consisting of citrate buffer, phosphate buffer, citrate-phosphate (Mcllvaine) buffer and acetate buffer.

5. The composition of claim 4, wherein the buffer is citrate buffer.

6. The composition of any one of claims 1 to 5, which also comprises an agent capable of improving muco-adhesion upon buccal administration.

7. The composition of claim 6, wherein the agent capable of improving muco-adhesion is an agent that gels by complexation with divalent cations present in the saliva on the surface of the mucous membranes of the mouth, in particular sodium alginate.

8. The composition of claim 6, which also comprises a poloxamer as a agent increasing gelling on contact with the mucous membranes of the buccal cavity, in particular the mucous membranes of the cheeks or the sublingual mucous membranes.

9. The composition of any one of claims 1 to 8, further containing at least one further component selected from the group consisting of aromas, flavors, dyes, sweetener and masking agent.

10. The composition of any one of claims 1 to 9, further containing a preservative.

11. The composition of any one of claims 1 to 10, which is packaged as a single unit dose, which contains from 0.1 to 2 mg atropine.

12. The composition of any one of claims 1 to 10, which is packaged in a kit further containing a syringe dose dropper adapted for viscous formulation.

13. The composition of any one of claims 1 to 10, which is packaged as a spray, wherein an amount from 0.1 to 2 mg atropine is delivered for each spray.

14. A method for preparing a composition according to any one of claims 1 to 13, comprising:
a) Preparing a solution of atropine in a buffer so as to obtain a solution with a pH between 3.5 and 4.5
b) Optionally adding aromas, flavors, dyes, sweetener and masking agent
c) Optionally adding an agent capable of improving muco-adhesion upon buccal administration
d) Optionally adding a preservative
e) Adding the thickening agent

15. A composition according to any one of claims 1 to 13 for use thereof for the treatment of sialorrhea, in particular second to neurological diseases.
